(19) Europäisches Patentamt
European Patent Office
Office européen des brevets

(11) Veröffentlichungsnummer: **0 275 390 B1**

(12) # EUROPÄISCHE PATENTSCHRIFT

(45) Veröffentlichungstag der Patentschrift: **22.04.92**

(51) Int. Cl.$^5$: **A61B 5/14**

(21) Anmeldenummer: **87116691.4**

(22) Anmeldetag: **12.11.87**

(54) **Implantierbarer elektrochemischer Sensor.**

(30) Priorität: **03.01.87 DE 3700119**

(43) Veröffentlichungstag der Anmeldung:
**27.07.88 Patentblatt 88/30**

(45) Bekanntmachung des Hinweises auf die Patenterteilung:
**22.04.92 Patentblatt 92/17**

(84) Benannte Vertragsstaaten:
**AT CH DE FR GB GR IT LI**

(56) Entgegenhaltungen:
**EP-A- 0 206 531**
**WO-A-86/04223**
**DE-A- 3 225 871**
**FR-A- 2 528 315**
**GB-A- 2 108 675**
**GB-A- 2 159 625**
**US-A- 4 253 456**

**Glycaemic Control in Pancreatectomized Dogs", M. Shichiri et al., Diabetologia (1983) 24: 179-184**

(73) Patentinhaber: **Institut für Diabetestechnologie Gemeinnützige Forschungs - und Entwicklungsgesellschaft mbH**
**Steinhövelstrasse 9**
**W-7900 Ulm(DE)**

(72) Erfinder: **Zier, Horst D.**
**Hauptstrasse 61**
**W-7341 Amstetten(DE)**
Erfinder: **Kerner, Wolfgang, Priv.-Doz. Dr.**
**Lerchenweg 14**
**W-7909 Dornstadt-Bollingen(DE)**
Erfinder: **Pfeiffer, Ernst F., Prof. Dr.Dr.h.c.mult.**
**Stauffenbergstrasse 34**
**W-7900 Ulm(DE)**

(74) Vertreter: **Wolf, Eckhard, Dr.-Ing.**
**Eugensplatz 5 Postfach 13 10 01**
**W-7000 Stuttgart 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft einen implantierbaren Sensor für amperometrische Messungen in Körperflüssigkeiten der im Oberbegriff des Anspruchs 1 angegebenen Gattung. Der Sensor ist vor allem für Blut- und Gewebezuckerkontrollen bei Diabetes-Patienten bestimmt und eignet sich für die Verwendung in einem tragbaren Diagnose- und/oder Therapiegerät.

Ein Patient, der an der Stoffwechselkrankheit Diabetes mellitus leidet, muß mehrmals täglich durch Spritzen von Insulin seinen Blutzuckerspiegel ausgleichen. Um sich vor Stoffwechselentgleisungen zu schützen, sind außerdem Blutzuckerkontrollen erforderlich. Die Blutzuckerkontrollen erfordern jedesmal Stiche in die Fingerbeere, was vor allem für Kleinkinder und Jugendliche eine große Belastung darstellt. Andererseits kann die ständig wechselnde Stoffwechsellage des Diabetikers zu den gefürchteten diabetischen Spätschäden führen, die vor allem auf Veränderungen der Gefäße und eine Verschlechterung der Sauerstoffversorgung einzelner Organe zurückzuführen sind.

Zur Erleichterung der Blutzuckerkontrolle werden im klinischen Bereich schon seit längerer Zeit mit Erfolg elektrochemische Enzymsensoren zur Blutglucosebestimmung in vitro eingesetzt. Die betreffenden Apparaturen sind in ihrem Aufbau jedoch noch recht aufwendig und schwer, so daß sie nur für die stationäre Behandlung in Betracht kommen.

Es wurde auch schon die Verwendung von inplantierbaren Enzymsensoren vorgeschlagen (Aufsatz M. Schichiri et al.: Glycaemic Control in Pancreatectomized Dogs with a Wearable Artificial Endocrine Pancreas", Diabetologia (1983) 24: 179 - 184), die in Verbindung mit tragbaren Meß- und Auswertegeräten zum Einsatz gebracht werden sollen. Ein bekannter nadelförmiger Glucosesensor dieser Art weist eine aus einem Platindraht bestehende Meßanode, eine aus Silber bestehende Referenzkathode, einen zwischen der Meßanode und der Referenzkathode angeordneten Glasisolator, eine auf der aktiven Elektrodenoberfläche angeordnete Enzymschicht aus immobilisierter Glucoseoxidase sowie eine die Enzymschicht abdeckende poröse Membran aus Polyurethan auf. Die bei einer Polarisationsspannung von etwa 600 mV gemessenen Diffusionsgrenzströme liegen bei Vorhandensein von $\beta$-D-Glucose in einer elektrolytischen Körperflüssigkeit bei einigen 100 $\mu$A bis zu einigen nA in linearer Abhängigkeit von der Glucosekonzentration. Bei in vivo-Messungen tritt jedoch mit der Zeit allmählich eine Drift zu niedrigeren Diffusionsgrenzströmen auf, die vermutlich auf Fibrin-Ablagerungen aus dem Gewebe und/oder Blut zurückzuführen sind. Diese Drift kann zwar durch Nach-kalibrierung in gewissen Zeitabständen kompensiert werden. Sofern diese Nachkalibrierung nicht automatisch erfolgen kann, treten hierbei jedoch Handhabungsschwierigkeiten auf, die einem breiten Einsatz der Sensoren entgegenstehen könnten. Dies gilt umso mehr, als die aus Silber bestehende und daher kostenbestimmende Bezugselektrode für einen Verbrauchsartikel noch zu hohe Materialkosten erfordert. Schließlich fehlen für den praktischen Einsatz der inplantierbaren Glucosesensoren die Datenerfassungs- und Auswertegeräte, die für den Transport an oder im Körper leicht und kompakt genug sind und die für einen langfristigen Batteriebetrieb geeignet sind.

Der Erfindung liegt die Aufgabe zugrunde, einen implantierbaren elektrochemischen Sensor zu schaffen, der mit geringem Materialkostenaufwand als Massenartikel für den breiten Einsatz herstellbar ist und trotzdem gute biokompatible und elektrochemische Eigenschaften aufweist.

Zur Lösung dieser Aufgabe wird die im Patentanspruch 1 angegebenen Merkmalskombination vorgeschlagen. Weitere vorteilhafte Ausgestaltungen und Weiterbildungen der Erfindung ergeben sich aus den Unteransprüchen.

Versuche mit Edelstahl als Bezugselektroden haben gezeigt, daß vor allem bei Vorhandensein eines Molybdänanteils von wenigen Gewichtsprozent sich bei der amperometrischen Messung mit Polarisationsspannungen zwischen 680 und 750 mV überraschend gut reproduzierbare Glucosegehaltsbestimmungen mit einer Linearität bis zu Werten um 800 mg/dl durchführen lassen. Der genannte Edelstahl hat sich im hohen Maße als biokompatibel erwiesen. Bei in vivo-Messungen im subcutanen Gewebe von Schafen wurde uber eine Meßdauer von 4 Tagen hinweg keine nennenswerte Abnahme in der Meßempfindlichkeit festgestellt: Die Drift lag bei verschiedenen Versuchen zwischen 11 und 15 % Empflindlichkeitsabnahme gegenüber dem Anfangswert. Ausgehend hiervon wird gemäß der Erfindung daher vorgeschlagen, daß die Bezugselektrode aus einem Edelstahl mit Molybdän als Legierungsbestandteil besteht. Als besonders vorteilhaft hat sich ein Edelstahl mit 16 bis 20 % Chrom-Anteil, 9 bis 13 % Nickel-Anteil, 1,5 bis 4,5 % Molybdän-Anteil und maximal 0,2 % Kohlenstoff-Anteil erwiesen, wobei gegebenenfalls noch Legierungsbestandteile Niob und/oder Titan mit einem Gewichtsanteil von weniger als 1 % vorhanden sein können.

Die Bezugselektrode ist zweckmäßig als dunne Kanüle ausgebildet, innerhalb der die Meßelektrode in einer Kunstharzmasse in axialer Ausrichtung mit einem zur Kanülenspitze weisenden und mit dem Enzym beschichten freien Ende eingebettet ist. Die Kanülenspitze sollte dabei mit einer für die Körperflüssigkeit und die darin gelöste Glucose sowie für Sauerstoff durchlässigen und für das Enzym undurchlässigen

Membran, vorzugsweise aus porösem Polyurethan, beschichtet sein.

Wie einleitend bereits ausgeführt wurde, kann es nach längerer Diabetesdauer zu Veränderungen der Gefäße und zu einem Defizit an Sauerstoffversorgung in den Organen und im Gewebe kommen. Eine Früherkennung dieses Sauerstoffdefizits kann zur Verhinderung von diabetischen Spätschäden beitragen. Weiter kann ein Sauerstoffmangel in der Gewebeflüssigkeit dazu führen, daß es zu systematischen Abweichungen bei der Glucosebestimmung kommt. Aus diesen Gründen besteht vor allem bei Diabetikern ein Bedarf dahingehend, daß neben der Blut- oder Gewebezuckermessung auch eine Überwachung der Sauerstoffversorgung von Gewebe und Organen durchgeführt wird.

Mit dem erfindungsgemäßen Sensor kann diese Messung durch einfaches Umpolen der Meßelektrode und der Bezugselektrode durchgeführt werden. Besonders vorteilhaft in diesem Zusammenhang ist es, wenn innerhalb der zylinderförmigen Bezugselektrode mindestens zwei zueinander parallel ausgerichtete Meßelektroden angeordnet sind, von denen nur eine mit dem Enzym beschichtet ist und die an eine gemeinsame, jedoch umpolbare Meßleitung oder an voneinander getrennte Meßleitungen angeschlossen sein können.

Eine besonders gute Trennung zwischen den elektrochemischen Vorgängen innerhalb des Sensors wird dadurch erreicht, daß mindestens zwei gegeneinander elektrisch isolierte Bezugselektroden vorgesehen sind, von denen je eine einer der Meßelektroden elektrochemisch zugeordnet ist. Die Bezugselektroden können dabei aus unterschiedlichen Werkstoffen bestehen, wobei die der Meßelektrode mit Enzymbeschichtung zugeordnete Bezugselektrode aus Edelstahl mit Molybdänbestandteil besteht, während die der Meßelektrode ohne Enzymbeschichtung zugeordnete Bezugselektrode aus Silber oder Silber/Silberchlorid bestehen kann. Die Bezugselektroden sind vorteilhafterweise als teilzylindrische Schalen ausgebildet, die sich zu einer rohrförmigen Kanüle ergänzen und an ihrer Naht mit einem isolierenden Kunstharz miteinander verbunden sind.

Die Meßelektroden und die Bezugselektroden werden paarweise mit den Eingängen mindestens eines Meßverstärkers verbunden. Verschiedene Gruppen von Meßelektroden und Bezugselektroden können dabei mit entgegengesetzt gepolter Polarisationsspannung beaufschlagt werden. Im Falle eines Kombinationssensors fur die Glucose- und Sauerstoffmessung weisen die mit Glucoseoxidase beschichtete Platin-Meßelektrode und die enzymfreie Platin-Meßelektrode gegenüber ihren Bezugselektroden aus Edelstahl bzw. Silber entgegengesetzt gepolte Polarisationsspannungen von vorzugsweise 600 bis 750 mV auf.

Der erfindungsgemäße implantierbare Sensor wird vorzugsweise in Verbindung mit einem Diagnosegerät zur Überwachung von Inhaltsstoffen in Körperflüssigkeiten, wie $\beta$-D-Glucose und/oder Sauerstoff in Blut oder Gewebeflüssigkeit, verwendet. Ein solches Gerät enthält einen mit dem Sensorsignal beaufschlagbaren Meßverstärker sowie eine einen A/D-Wandler enthaltende Schaltung zur Auswertung des Ausgangssignals des Meßverstärkers. Unter der Voraussetzung, daß zwischen dem Meßstrom und der zu messenden Stoffkonzentration ein linearer Zusammenhang besteht, ist eine einfache Kalibrierung dadurch möglich, daß am Eingang des A/D-Wandlers ein Referenzpotential unter Anpassung an vorgegebene Werte eines Testmediums stufenlos einstellbar ist. Der auf diese Weise geeichte digitale Meßwert kann in vorgegebenen Zeitabständen in einem Schreiblese-Speicher abgespeichert werden. Um die Stoffwechsellage des Patienten zu bestimmen, können diese Werte in größeren Zeitabständen über eine Schnittstelle ausgelesen und auf einen Drucker und/oder externen Computer übertragen werden. Wenn die Meß- und Speicherschaltung als batteriebetriebene, in stromsparender CMOS-Technik ausgeführte Anordnung ausgebildet ist, kann sie leicht in einem transportablen Gerät untergebracht werden, das am Körper des Patienten mitgeführt werden kann. An einer LCD-Anzeige können die momentanen Meß- und Betriebswerte abgelesen werden.

In einer weiteren vorteilhaften Ausgestaltung dieses Geräts kann noch ein Mikroprozessor vorgesehen werden, der ein Programm zur Ansteuerung einer Insulinpumpe nach Maßgabe der Abweichung der Blut- oder Gewebezuckermeßwerte von mindestens einem Sollwert oder Sollwertverlauf enthält. Die vom Programm ermittelten Insulinmengenwerte können zusammen mit den Glucosemeßwerten im Schreiblese-Speicher abgespeichert und über die Schnittstelle abgerufen werden. Damit erhält man ein tragbares Gerät, das zur automatischen Regelung des Glucose-Stoffwechsels des Patienten geeignet ist. Aufgrund der vollständigen Datenerfassung wird außerdem die Stoffwechsellage des Patienten in dichten Zeitabständen überwacht, so daß etwaige Störungen nachvollzogen und erforderlichenfalls vom Arzt korrigiert oder behandelt werden können.

Im folgenden wird die Erfindung anhand der Zeichnung näher erläutert. Es zeigen

- Fig. 1 einen Enzymsensor mit einer Meßelektrode und einer Bezugselektrode in senkrecht geschnittener Darstellung;
- Fig. 2 einen Kombinationssensor mit zwei Meßelektroden und einer gemeinsamen Bezugselektrode in senkrecht geschnittener Darstellung;
- Fig. 3 einen Kombinationssensor mit zwei getrennten Teilzellen in senkrecht geschnittener Darstellung;

Fig. 4 eine Meßschaltung für den Enzymsensor nach Fig. 1;

Fig. 5 eine Meßschaltung für den Kombinationssensor nach Fig. 2;

Fig. 6 eine Meßschaltung für den Kombinationssensor nach Fig. 3;

Fig. 7 ein Blockschaltbild eines Diagnosegeräts;

Fig. 8 ein Blockschaltbild eines automatischen Diagnoseund Therapiegeräts.

Die in den Fig. 1 bis 3 in schematischer Weise vergrößert dargestellten elektrochemischen Enzymsensoren 1 sind für in vivo-Messungen im menschlichen und tierischen Körper bestimmt. Sie bestehen im wesentlichen aus einem dünnen zylindrischen Rohr 10 aus Metall, das eine isolierende Epoxidharzfüllung 12 sowie eine oder zwei axial ausgerichtete Meßelektroden 14,14′ enthält. Der metallische Rohrmantel bildet zugleich die Bezugselektrode 11,11′ des elektrochemischen Sensors.

Die aktive Meßstelle 16 des Sensors befindet sich an dessen Stirnfläche, an der die Meßelektrode 14 aus der Epoxidharz-Isolierung 12 herausragt und von einer halbdurchlässigen, biokompatiblen porösen Membran 18 und einem innerhalb der Membran eingeschlossenen Enzym 20 beschichtet ist.

Die Meßelektroden 14,14′ bestehen vorzugsweise aus Platin oder Gold, während die Bezugselektroden 11 der Enzymzellen aus Edelstahl mit Molybdän als Legierungsbestandteil bestehen. Im Falle der Fig. 3 sind zwei als zylindrische Halbschalen ausgebildete Bezugselektroden 11,11′ vorgesehen, die mit Hilfe einer nicht dargestellten Kunststoff-Isolierung unter Bildung eines Rohrs 10 miteinander verbunden sind. Die zur Enzymzelle gehörende Bezugselektrode 11 besteht wiederum aus Edelstahl mit Molybdänanteil, während die zur enzymfreien Teilzelle gehörende Bezugselektrode 11 bevorzugt aus Silber besteht.

Der nadelförmige Sensor 1 hat einen Durchmesser von ca. 0,5 - 1 mm, wobei die Meßelektroden 14,14′ einen Durchmesser von 0,15 bis 0,2 mm aufweisen. Das von dem Platindraht oder den Platindrähten durchsetzte Röhrchen 10 wird mit noch flüssigem, mit einem Härter versetzten Epoxidharz 12 ausgefüllt und in seiner Lage festgehalten, bis das Harz ausgehärtet ist. Sodann wird das stirnseitige Ende 16 vom austretenden Epoxidharz befreit und unter Freilegung der Drahtenden abgeschliffen. Anschließend wird die Sensorspitze in eine Suspension von Glucoseoxidase 20 in Ethanol-Aceton eingetaucht und luftgetrocknet. Durch Eintauchen in eine Lösung von Polyurethan in Tetrahydrofuran und anschließendes Lufttrocknen wird die semipermeable PUR-Membran 18 gebildet. Bei den in den Fig. 2 und 3 gezeigten Kombinationssensoren wird eine der Platinelektroden 14′ und ein Teil der Bezugselektrode 11′ nach dem Beschichten mit dem Enzym wieder von diesem befreit oder vor dem Beschichten mit einer anschließend zu entfernenden Abdeckung versehen, so daß ein enzymfreier Bereich 22 entsteht.

In den Fig. 4 bis 6 sind die mit FET-Operationsverstärkern 30 bestückten Meßschaltungen 3 gezeigt, an die die Sensoren nach Fig. 1 bis 3 wie folgt anschließbar sind:

Die Meßschaltung nach Fig. 4 ist für den Anschluß der Enzymelektrode nach Fig. 1 bestimmt. Die Platinanode 14 (+) wird an den invertierenden Eingang des Operationsverstärkers 30 gelegt, während die Edelstahlkathode 11 (-) mit einer an der Spannungsteilerschaltung 32 über ein Potentiometer 34 einstellbaren negativen Vorspannung an den nichtinvertierenden Eingang des OP-Verstärkers 30 angelegt wird. Die eingestellte Vorspannung wird über eine Z-Diode 36 stabilisiert. Das Meßsignal wird in einer am Ausgang des Meßverstärkers 3 angeschlossenen Auswerteschaltung 38 in der unten noch näher erläuterten Weise weiterverarbeitet.

Bei der Meßdurchführung dient die Körperflüssigkeit als Elektrolyt, in den der elektrochemische Sensor beispielsweise durch Implantieren eingetaucht ist. Im Glucose-Sensor, der Glucoseoxidase als Enzym 20 enthält, wird in der Körperflüssigkeit vorhandene $\beta$-D-Glucose im Beisein von Sauerstoff in D-Glucoselactone unter Freisetzung von $H_2O_2$ oxidiert:

$$\beta\text{-D-Glucose} + O_2 \xrightarrow{\text{GOD}} \text{D-Glucoselactone} + H_2O_2$$

Die Bildungsrate von $H_2O_2$ kann in der elektrochemischen Zelle als Diffusionsgrenzstrom aufgrund der folgenden Reaktion an der Platinanode gemessen werden:

$$H_2O_2 \xrightarrow{\text{Pt}} O_2 + 2\,H^+ + 2\,e^-.$$

In einer Zelle mit Platinanode und Edelstahlkathode läuft die angegebene Reaktion bevorzugt bei einer Polarisationsspannung von 680 bis 750 mV ab. Der meßbare Diffusionsgrenzstrom ist hierbei in weiten Grenzen proportional zur Glucosekonzentration in der Körperflüssigkeit. Der im Gewebe oder im Blut vorhandene Sauerstoff reicht für den Reaktionsablauf aus, zumal der bei der Glucose-Umwandlung verbrauchte Sauerstoff bei der Umwandlung von $H_2O_2$ an der Pt-Anode (11) zurückgewonnen und zumindest teilweise wieder in die Körperflüssigkeit zurückgeführt wird.

Grundsätzlich ist es möglich, den in Fig. 1 gezeigten Sensor in Verbindung mit einer der Meßschaltungen auch mit entgegengesetzter Polarsationsspannung, also mit dem Platindraht als Kathode und dem Edelstahlrohr als Anode, zu betreiben. Damit erhält man an der Meßelektrode eine Reduktion des in der Körperflüssigkeit enthaltenen Sauerstoffs zunächst zu $H_2O_2$ und anschließend zu $H_2O$ nach folgenden Reaktionsgleichungen:

$$O_2 + 2\,H^+ + 2\,e^- \xrightarrow{Pt} H_2O_2$$

$$H_2O_2 + 2\,H^+ + 2\,e^- \xrightarrow{Pt} 2\,H_2O$$

Die Plateauspannung für die Messung des Diffusionsgrenzstroms dieser beiden Reaktionsstufen liegt bei -600 bis -700 mV. Zur Messung des Sauerstoffpartialdrucks in der Körperflüssigkeit wird also der Differenzgrenzstrom bei konstanter Spannung zwischen Meßkathode und Bezugsanode als Maß für die in der Zeiteinheit die Kathode erreichenden $O_2$-Molekule genutzt. Der beschriebene Sensor nach Fig. 1 bis 3 kann also durch einfache Umpolung der Meßelektrode und der Bezugselektrode zur Messung und Überwachung zweier verschiedener Meßsubstanzen, nämlich $\beta$-D-Glucose und Sauerstoff, verwendet werden.

Der in Fig. 1 gezeigte Sensor ist als Kombinationssensor allerdings insofern noch nicht optimal, als im Falle der Beschaltung als Sauerstoffsensor der Sauerstoff durch die Enzymschicht 20 hindurchdiffundieren müßte, was zu einer Erniedrigung des Diffusionsgrenzstroms und damit der Empfindlichkeit führen würde. Um diesen Nachteil zu vermeiden, sind bei den Sensoren nach Fig. 2 und 3 zwei Meßelektroden 14,14′ vorgesehen, von denen nur eine in ihrem aktiven Bereich mit dem Enzymreaktor 20 beschichtet ist, während die andere dort enzymfrei ist. Die Enzym-Teilzelle weist somit bei entsprechender Beschaltung in den Meßschaltungen in Fig. 5 und 6 eine als Anode beschaltete Meßelektrode 14 aus Platin und eine als Kathode beschaltete Bezugselektrode 11 aus Edelstahl auf, während die Sauerstoffteilzelle mit einer als Kathode beschalteten Platinmeßelektrode 14′ und einer als Anode beschalteten Bezugselektrode 11 besteht. Im Falle der Fig. 2 ist eine gemeinsame Bezugselektrode 11,11′ aus Edelstahl vorgesehen, die von Fall zu Fall über den Umschalter 40 der Meßschaltung nach Fig. 5 als Kathode und Anode beschaltet wird, während im Falle der Fig. 3 zwei gegeneinander elektrisch isolierte, aus zylindrischen Halbschalten bestehende Bezugselektroden 11,11′ vorgesehen sind, von denen die der Enzym-Teilzelle als Kathode 11 beschaltet ist und vorzugsweise aus Edelstahl besteht, während die der $O_2$-Teilzelle als Anode 11′ beschaltet ist und vorzugsweise aus Silber besteht.

In der Meßschaltung nach Fig. 6 werden die Elektroden 11,11′,14,14′ der beiden Teilzellen an voneinander unabhängige Meßkanäle 30,30′ gelegt, die an ihren Ausgängen über einen Umschalter 42 wahlweise mit der Auswerteschaltung 38 verbunden werden können. Damit wird beim Umschalten eine Umpolarisation innerhalb der Zellen vermieden, so daß die Messungen an den beiden Teilzellen im raschen Wechsel durchgeführt werden können.

Die beschriebenen Glucosesensoren mit ihren Meßschaltungen können in Verbindung mit einem tragbaren Diagnosegerät (Fig. 7) oder in einem kombinierten Diagnose- und Therapiegerät (Fig. 8) als Auswerteschaltung 38 verwendet werden.

Die Tatsache, daß ein linearer Zusammenhang zwischen den im Elektrolyten vorhandenen Stoffkonzentrationen (Glucose bzw. Sauerstoff) und dem gemessenen Diffusionsgrenzstrom besteht, kann dazu genutzt werden, daß die Kalibrierung der Geräte relativ einfach am analogen Meßverstärker durch stufenlose Verstellung des Nullpunkts (zero) und des Verstärkungsgrads (full scale) vorgenommen werden kann.

Das in Fig. 7 in einem Blockschaltbild gezeigte Diagnosegerät ist im wesentlichen wie folgt aufgebaut:

Die am Ausgang des Meßverstärkers 3 anstehenden Analogdaten werden in einem Analog/Digital-Wandler 50 in Digitalwerte umgewandelt und in dieser Form in einer LCD-Anzeige 52 angezeigt und in einem Parallelspeicher 54 zwischengespeichert. Die zwischengespeicherten Daten werden in bestimmten Zeitintervallen, die über eine Quarzuhr 56 und ein extern einstellbares Zeitwahlgerät 58 bestimmt werden, in einen Schreiblese-Speicher 60 eingeschrieben. Dieser Vorgang wird über ein Schreibsteuerwerk 62 ausgelöst, das sowohl ein Adreßzählwerk 64 als auch den Schreib- und den Chip-Select-Eingang des Schreiblese-Speichers 60 ansteuert. Der als stromsparender CMOS-RAM ausgebildete Schreiblese-Speicher 60 ist batteriegepuffert (66), so daß die gespeicherten Daten auch bei abgeschaltetem Gerät erhalten bleiben. Der Inhalt des Schreiblese-Speichers kann über eine Auslesesteuerung 68, die über eine extern anschließbare Schnittstelle 70 ansteuerbar ist, zu einem Ausgangsregister ausgelesen und von dort auf einen externen Computer oder unmittelbar auf einen Drucker übertragen werden. Unter Verwendung moderner Halbleiterbauteile kann die gesamte Schaltung auf einer einzigen Platine untergebracht werden, so daß zur Meßdatenerfassung und -speicherung ein kleines leichtes Gerät zur Verfügung gestellt werden kann, das ohne den Bewegungsablauf zu stören unmittelbaren am Körper des Patienten angebracht werden kann. Bei weitergehender Miniaturisierung der Bauteile läßt sich das Gerät in seinen Abmessungen so weit reduzieren, daß es ähnlich wie eine Armbanduhr am Arm getragen werden kann.

Das in Fig. 8 in einem schematischen Blockschaltbild gezeigte Diagnose- und Therapiegerät ist eine Weiterbildung des Diagnosegeräts nach Fig. 7. Es enthält zusätzlich einen Mikroprozessor 80 zur Auswertung der ankommenden digitalen Meßwerte. Um den Diabetes-Patienten vor gefährlichen Unterzuckerungen und Überzuckerungen zu schützen, wird bei Unterschreitung eines Blutzuckerwerts von 40 mg/dl und bei Überschreiten eines Werts von 300 mg/dl über den Mikroprozessor 80 ein Alarmsignal ausgelöst. Weiter kann über ein Mikroprozessor-Programm nach Maßgabe der gemessenen Blut- oder Gewebezuckerwerte und unter Berücksichtung verschiedener vom Arzt einzugebender Patientenparameter eine Insulinpumpe 82 angesteuert werden. Die Insulinpumpe führt über einen Schrittmotor jeweils die vom Mikroprozessor errechnete Insulinmenge über einen im Bauchgewebe liegenden Nadelkatheder dem Patienten zu.

Die aktuellen Glucosewerte und die von der Insulinpumpe 82 abgegebenen Insulinmengen, werden in dem Schreiblese-Speicher in vorgegebenen Zeitabständen gespeichert und können über ein extern anzuschließendes Interface 70 von Zeit zu Zeit aus dem Speicher ausgelesen und ausgedruckt oder in einem Computer zur Überprüfung der Stoffwechseleinstellung des Patienten weiter ausgewertet werden.

## Patentansprüche

1. Implantierbarer Sensor für amperometrische Messungen in Körperflüssigkeiten, wie Blut oder Gewebeflüssigkeit, mit einer auf vorzugsweise als Platin- oder Golddraht ausgebildeten Meßelektrode, einer die Meßelektrode rohrförmig umfassenden Bezugselektrode sowie einer auf den Elektroden angeordneten Schicht aus einem immobilisierten Enzym, wie Glucoseoxidase, **dadurch gekennzeichnet,** daß die Bezugselektrode (11) aus einem Edelstahl mit Molybdän als Legierungsbestandteil besteht.

2. Sensor nach Anspruch 1, **dadurch gekennzeichnet,** daß die Bezugselektrode (11) aus einem Stahl mit 16 bis 20 % Chrom-Anteil, 9 bis 13 % Nickel-Anteil, 1,5 bis 4,5 % Molybdänanteil und maximal 0,2 % Kohlenstoffanteil ist.

3. Sensor nach Anspruch 2, **dadurch gekennzenchnet,** daß der Molybdänanteil 1,8 bis 2,3 % beträgt.

4. Sensor nach einem der Ansprüche 1 bis 3, **dadurch gekennzeichnet,** daß die Bezugselektrode (11) als weitere Legierungsbestandteile Niob und/oder Titan mit einem Gewichtsanteil von weniger als 1 % enthält.

5. Sensor nach einem der Ansprüche 1 bis 4, **dadurch gekennzeichnet,** daß die Bezugselektrode (11,11′) als Kanüle (10) ausgebildet ist, innerhalb der die Meßelektrode (14,14′) in einer Kunstharzmasse (12) in axialer Ausrichtung mit einem zur Kanülenspitze (16) weisenden und mit dem Enzym (20) beschichteten freien Ende eingebettet ist, und daß die Kanülenspitze mit einer für die Körperflüssigkeit und die darin gelöste Glucose sowie Sauerstoff durchlässigen und für das Enzym (20) undurchlässigen Membran (18), vorzugsweise aus porösem Polyurethan, beschichtet ist.

6. Sensor nach Anspruch 5, **dadurch gekennzeichnet,** daß in der Kanüle (10) mindestens zwei zueinander parallel ausgerichtete Meßelektroden (14,14′) angeordnet sind.

7. Sensor nach Anspruch 6, **dadurch gekennzeichnet,** daß nur eine oder ein Teil der Meßelektroden

(14) mit dem Enzym (20) beschichtet ist.

**8.** Sensor nach Anspruch 6 oder 7, **dadurch gekennzeichnet,** daß die Meßelektroden (14,14′) an eine gemeinsame Meßleitung angeschlossen sind.

**9.** Sensor nach Anspruch 6 oder 7, **dadurch gekennzeichnet,** daß mindestens zwei Gruppen der Meßelektroden (14,14′) an voneinander getrennte Meßleitungen angeschlossen sind.

**10.** Sensor nach einem der Ansprüche 6 bis 9, **dadurch gekennzeichnet,** daß die Kanülenspitze (16) mit Meßelektrode (14,14′) und zumindest ein Teil der Mantelfläche der die Bezugselektrode (11,11′) bildenden Kanüle (10) mit einer zusammenhängenden, Sauerstoff-permeablen Membran (18) überzogen sind.

**11.** Sensor nach einem der Ansprüche 6 bis 10, **dadurch gekennzeichnet,** daß mindestens zwei gegeneinander elektrisch isolierte Bezugselektroden (11,11′) vorgesehen sind, von denen je eine einer Meßelektrode (11,11′) oder einer Meßelektrodengruppe elektrochemisch zugeordnet ist.

**12.** Sensor nach Anspruch 11, **dadurch gekennzeichnet,** daß mindestens eine Bezugselektrode (11) aus Edelstahl mit Molybdän als Legierungsbestandteil besteht und daß mindestens eine einer Meßelektrode (14′) ohne Enzymbeschichtung zugeordnete Bezugselektrode (11′) aus Silber oder Silber/Silberchlorid besteht.

**13.** Sensor nach Anspruch 11 oder 12, **dadurch gekennzeichnet,** daß die Bezugselektroden (11,11′) aus teilzylindrischen Schalen bestehten, die sich zu einer rohrförmigen Kanüle (10) ergänzen und an ihrer Naht mit einem isolierenden Kunstharz miteinander verbunden sind.

**14.** Sensor nach einem der Ansprüche 11 bis 13, **dadurch gekennzeichnet,** daß die Meßelektroden (14,14′) und die Bezugselektroden (11,11′) paarweise an die Eingänge mindestens eines Meßverstärkers (3) anlegbar sind.

**15.** Sensor nach Anspruch 14, **dadurch gekennzeichnet,** daß verschiedene Gruppen von Meßelektroden (14,14′) und Bezugselektroden (11,11′) mit entgegengesetzt gepolter Polarisationsspannung beaufschlagbar sind.

**16.** Sensor nach Anspruch 15, **dadurch gekennzeichnet,** daß die entgegengesetzt gepolten Gruppen von Meßelektroden (14,14′) und Bezugselektroden (11,11′) über einen die Polarisationsspannung umpolenden Umschalter (40) an die FET-Eingänge eines Meßverstärkers (3) anlegbar sind.

**17.** Sensor nach Anspruch 15, **dadurch gekennzeichnet,** daß die entgegengesetzt gepolten Gruppen von Meßelektroden (14,14′) und Bezugselektroden (11,11′) an die FET-Eingänge je eines Meßverstärkers (3,3′) anlegbar sind, deren Ausgänge umschaltbar an eine Auswerteschaltung (38) anlegbar sind.

**18.** Sensor nach einem der Ansprüche 6 bis 17, **dadurch gekennzeichnet,** daß die enzymbeschichtete Meßelektrode (14) mit einer positiven und die enzymfreie Meßelektrode (14′) mit einer negativen Polarisationsspannung gegenüber der zugehörigen Bezugselektrode (11,11′) beaufschlagbar ist.

**19.** Sensor nach Anspruch 18, **dadurch gekennzeichnet,** daß im Falle eines Kombinationssensors (1) fur die Glucose- und Sauerstoffmessung die mit Glucoseoxidase (20) beschichtete Platin-Meßelektrode (14) und die enzymfreie Platin-Meßelektrode (14′) gegenüber ihren Bezugselektroden (11,11′) aus Edelstahl und/oder Silber entgegengesetzt gepolte Polarisationsspannungen von 600 bis 750 mV aufweisen.

**20.** Gerät zur in vivo-Messung und Überwachung von Inhaltsstoffen in Körperflüssigkeiten, wie $\beta$-D-Glucose und/oder Sauerstoff in Blut oder Gewebeflüssigkeit, mit einem implantierbaren Sensor nach einem der Ansprüche 1 bis 19, einem mit dem Sensorsignal beaufschlagbaren Meßverstärker sowie einer einen A/D-Wandler enthaltenden Schaltung zur Auswertung des Ausgangssignals des Meßverstärkers, **dadurch gekennzeichnet,** daß am Eingang des A/D-Wandlers (50) mindestens ein Referenzpotential zur Kalibrierung des analogen Ausgangssignals des Meßverstärkers (3) unter Anpassung an vorgegebene

Werte eines Testmediums stufenlos einstellbar ist, daß die auf diese Weise geeichten digitalen Meßwerte in vorgegebenen Zeitabständen in einem Schreiblese-Speicher (60) abspeicherbar sind, daß der Inhalt des Schreiblese-Speichers (60) in größeren Zeitabständen über eine Schnittstelle (70) auslesbar und auf einen Drucker und/oder einen externen Computer übertragbar ist, und daß die Meß- und Speicherschaltung als batteriebetriebene, in stromsparender CMOS-Technik ausgeführte transportable Anordnung ausgebildet ist.

**21.** Gerät nach Anspruch 20, **dadurch gekennzeichnet,** daß zusätzlich eine LCD-Anzeige (52) zur Darstellung der momentanen Meß- und Betriebswerte vorgesehen ist.

**22.** Gerät nach Anspruch 20 oder 21, **dadurch gekennzeichnet,** daß die mit einer Vorspannung beaufschlagbaren und mit den Elektroden (11,11′,14,14′) des elektrochemischen Sensors (1) verbindbaren Eingangsanschlüsse des Meßverstärkers (3) umpolbar sind.

**23.** Gerät nach Anspruch 20 oder 21, **dadurch gekennzeichnet,** daß zwei mit verschiedenen Teilzellen des elektrochemischen Sensors verbundene Meßverstärker (3,3′) vorgesehen sind, deren Eingänge unabhängig voneinander mit einer einstellbaren Vorspannung beaufschlagt sind und deren Ausgänge wahlweise mit dem Analogeingang des A/D-Wandlers (50) verbindbar sind.

**24.** Gerät nach einem der Ansprüche 20 bis 23, **dadurch gekennzeichnet,** daß die Auswerteschaltung (38) einen mit den Meß- und/oder Betriebswerten des Geräts beaufschlagbaren Mikroprozessor (80) enthält, mit dem Alarmsignale und/oder die Zufuhr pharmazeutischer Wirkstoffe nach Maßgabe der Übereinstimmung und/oder Abweichung der augenblicklichen Meßwerte mit bzw. von mindestens einem Sollwert und/oder mindestens einem Sollwertverlauf und/oder nach Maßgabe der Betriebswerte auslösbar sind.

**25.** Gerät nach Anspruch 24, **dadurch gekennzeichnet,** daß der Mikroprozessor (80) ein Programm zur Ansteuerung einer Insulinpumpe (82) nach Maßgabe der Abweichung der gemessenen Blut- oder Gewebezuckerwerte von mindestens einem Sollwert oder Sollwertverlauf enthält und daß die vom Programm ermittelten Insulinmengenwerte zusammen mit den Glucose-Meßwerten im Schreiblese-Speicher (60) abspeicherbar und von der Schnittstelle abrufbar sind.

**Claims**

**1.** Implantable sensor for amperometric measuring in body fluids, such as blood or tissue liquid, by means of a measuring electrode which is preferably made of platinum wire or gold wire, a reference electrode which tubularly surrounds the measuring electrode, and a layer of an immobilising enzyme, such as glucoseoxidase, arranged on the electrodes, **characterised in that** the reference electrode (11) is made of a high-quality steel with molybden as an alloy portion.

**2.** Sensor according to claim 1, **characterised in that** the reference electrode (11) is made of a steel with between 16 and 20% chromium content, between 9 and 13% nickel content, between 1.5 and 4.5% molybden content and maximal 0.2% carbon content.

**3.** Sensor according to claim 2, **characterised in that** the molybden content is between 1.8 and 2.3%.

**4.** Sensor according to one of claims 1 to 3, **characterised in that** the reference electrode (11) includes niob and/or titanium with a weight content of less than 1% as a further alloy portion.

**5.** Sensor according to one of claims 1 to 4, **characterised in that** the reference electrode (11, 11') is arranged as a cannula (10), in which the measuring electrode (14, 14') is embedded in a synthetic resin mass (12) in axial alignment with a free end which is oriented towards the cannula tip (16) and coated With the enzyme (20), and that the cannula tip is coated with a diaphragm (18), which is permeable by the body liquid and therein dissolved glucose and oxygen and by the enzyme (20) and preferably made of porous polyurethane.

**6.** Sensor according to claim 5, **characterised in that** at least two measuring electrodes (14, 14') are arranged parallel to each other in the cannula (10).

7. Sensor according to claim 6, **characterised in that** only one, or a portion of the measuring electrodes (14) is covered by the enzyme (20).

8. Sensor according to claim 6 or 7, **characterised in that** the measuring electrodes (14, 14') are connected to a common measuring line.

9. Sensor according to claim 6 or 7, **characterised in that** at least two groups of measuring electrodes (14, 14') are connected to separated measuring lines.

10. Sensor according to one of claims 6 to 9, **characterised in that** the cannula tip (16) with measuring electrode (14, 14') and at least part of the covering surface of the cannulas (10) forming the reference electrode (11, 11') are covered in a continuous oxygen-permeable diaphragm (18).

11. Sensor according to one of claims 6 to 10, **characterised in that** at least two reference electrodes (11, 11') which are electrically insulated from each other are provided, of which one is electro-chemically associated with a measuring electrode (11, 11') or a measuring-electrode group.

12. Sensor according to claim 11, **characterised in that** at least one reference electrode (11) is made of high-quality steel with molybden as an alloy portion, and that at least one reference electrode (11') which is associated with a measuring electrode (14') without enzyme coating is made of silver or silver/silverchloride.

13. Sensor according to claim 11 or 12, **characterised in that** the reference electrodes (11, 11') consist at part-cylindrical dishes which form a tubular cannula (10) and which are joined at their seam by means of an insulating synthetic resin.

14. Sensor according to one of claims 11 to 13, **characterized in that** the measuring electrodes (14, 14') and the reference electrodes (11, 11') are connected in pairs to the input of at least one measuring amplifier (3).

15. Sensor according to claim 14, **characterised in that** different groups of measuring electrodes (14, 14') and reference electrodes (11, 11') are loaded with oppositely polarised polarisation voltage.

16. Sensor according to claim 15, **characterised in that** the oppositely polarised groups of measuring electrodes (14, 14') and reference electrodes (11, 11') are connected to the FET inputs of a measuring amplifier (3) via a switch (40) which repolarises the polarisation voltage.

17. Sensor according to claim 15, **characterised in that** the oppositely polarised groups of measuring electrodes (14, 14') and reference electrodes (11, 11') are connected to the FET inputs of a respective measuring amplifier (3, 3') the outputs of which can be switched over to an evaluation circuit (38).

18. Sensor according to one of claims 6 to 17, **characterised in that** the enzyme-coated measuring electrode (14) is loaded with a positive polarisation voltage, and the enzyme-free measuring electrode (14') with a negative polarisation voltage relative to the associated reference electrode (11, 11').

19. Sensor according to claim 18, **characterised in that,** in the case of a combination sensor (1) for glucose and oxygen measuring, the platinum measuring electrode (14) which is coated with glucoseoxidase (20) and the enzyme-free platinum measuring electrode (14') have opposite their reference electrodes (11, 11') of high-quality steel and/or silver oppositely polarised polarisation voltages between 600 and 750 mV.

20. Apparatus for in-vivo measuring and checking of content matters in body liquids, such as $\beta$-D-glucose and/or oxygen in blood or tissue liquid, with an implantable sensor according to one of claims 1 to 19, a measuring amplifier to be loaded with the sensor signal, and a circuit comprising an A/D converter for the evaluation of the output signals of the measuring amplifier, **characterised in that** at the input of the A/D converter (50) at least one reference potential for the calibration of the analog output signal of the measuring amplifier (3) is infinitely adjustable in line with specified values of a test medium, that thus calibrated digital measuring values are stored in specified time intervals in a write-read memory

(60), that the contents of the write-read memory (60) is read at larger time intervals via an interface (70) and transferable to a Printer and/or an external computer, and that the measuring and memory circuit is a battery-backed transportable arrangement in current-saving CMOS technique.

**21.** Apparatus according to claim 20, **characterised in that** an LCD indicator (52) for indicating instantaneous measuring and operational values is additionally provided.

**22.** Apparatus according to claim 20 or 21, **characterised in that** the input connections of the measuring amplifier (3), which are biassed and connected with the electrodes (11, 11', 14, 14') of the elctrochemical sensor (1), can be repolarised.

**23.** Apparatus according to claim 20 or 21, **characterised in that** two measuring amplifiers (3, 3') are provided, which are connected with different cell portions of the electro-chemical sensor and the inputs of which are loaded independent from each other with an adjustable bias, and the outlets of which are selectively connectable with the analog input of the A/D converter (50).

**24.** Apparatus according to one of claims 20 to 23, **characterised in that** the evaluation circuit (38) comprises a microprocessor (80), which is loaded with the measuring and/or operational values of the device and by which alarm signals and/air the feed of pharmaceutical substances can be actuated in agreement with and/or deviation of instantaneous measuring values with, or of at least one nominal value and/or at least one nominal value course and/or in agreement with the operational values,

**25.** Apparatus according to claim 24, **characterised in that** the microprocessor (80) includes a program for the control of an insulin pump (83) in agreement with the deviation of the measured blood or tissue sugar values from at least one nominal value or nominal value course, and that the quantitative insulin values determined by the program are stored with the glucose measuring values in the write-read memory (60) and accessible via the interface.

**Revendications**

**1.** Capteur à implanter pour des mesures ampérométriques dans des liquides organiques, tels que sang ou liquide tissulaire, avec une électrode de mesure, réalisée de préférence sous forme de fil de platine ou d'or, une électrode de référence, enveloppant en forme de tube l'électrode de mesure, et une couche d'enzyme immobilisée, telle que glucose oxydase, disposée sur les électrodes, caractérisé en ce que l'électrode de référence (11) se compose d'acier spécial, avec du molybdène comme élément d'alliage.

**2.** Capteur suivant la revendication 1, caractérisé en ce que l'électrode de référence (11) est en acier, avec 16 à 20% de chrome, 9 à 13% de nickel, 1,5 à 4,5% de molybdène, et 0,2% maximum de carbone.

**3.** Capteur suivant la revendication 2, caractérisé par une part de molybdène de 1,8 à 2,3%.

**4.** Capteur suivant l'une quelconque des revendications 1 à 3, caractérisé en ce que l'électrode de référence (11) comporte, comme autres éléments d'alliage, du niobium et/ou titane, avec un pourcentage en poids inférieur à 1%.

**5.** Capteur suivant l'une quelconque des revendications 1 à 4, caractérisé en ce que l'électrode de référence (11, 11') est réalisée sous forme de canule (10), à l'intérieur de laquelle l'électrode de mesure (14, 14'), orientée dans le sens axial, est noyée dans une masse de résine synthétique (12), avec une extrémité libre dirigée vers la pointe (16) de la canule et enduite de l'enzyme (20), et en ce que la pointe de la canule est revêtue d'une membrane (18), en polyuréthanne poreux de préférence, perméable au liquide organique, au glucose dissous dans ce dernier, et à l'oxygène, et imperméable à l'enzyme (20).

**6.** Capteur suivant la revendication 5, caractérisé en ce que deux électrodes de mesure (14, 14') au moins, parallèles entre elles, sont disposées dans la canule (10).

7. Capteur suivant la revendication 6, caractérisé en ce qu'une seule électrode, ou une partie des électrodes de mesure (14), est enduite de l enzyme (20).

8. Capteur suivant l'une des revendications 6 et 7, caractérisé en ce que les électrodes de mesure (14, 14') sont raccordées à une ligne de mesure commune.

9. Capteur suivant l'une des revendications 6 et 7, caractérisé en ce que deux groupes au moins d'électrodes de mesure (14, 14') sont raccordés à des lignes de mesure distinctes l'une de l'autre.

10. Capteur suivant l'une quelconque des revendications 6 à 9, caractérisé en ce que la pointe (16) de la canule avec l'électrode de mesure (14, 14'), et une partie du moins de l'enveloppe de la canule (10), formant l'électrode de référence (11, 11'), sont revêtues d'une membrane (18) cohérente, perméable à l'oxygène.

11. Capteur suivant l'une quelconque des revendications 6 à 10, caractérisé en ce que deux électrodes de référence (11, 11') au moins, isolées électriquement l'une par rapport à l'autre, sont prévues, dont chacune est associée électrochimiquement à une électrode de mesure (11, 11'), ou à un groupe d'électrodes de mesure.

12. Capteur suivant la revendication 11, caractérisé en ce qu'une électrode de référence (11) au moins se compose d'acier spécial, avec du molybdène comme élément d'alliage, et en ce qu'une électrode de référence (11') au moins, associée à une électrode de mesure (14') sans enduction d'enzyme, se compose d'argent ou d argent/chlorure d'argent.

13. Capteur suivant l'une des revendications 11 et 12, caractérisé en ce que les électrodes de référence (11, 11') se composent de coques semi-cylindriques, qui se complètent pour former une canule tubulaire (10), et sont assemblées sur leur joint par une résine synthétique isolante.

14. Capteur suivant l'une quelconque des revendications 11 à 13, caractérisé en ce que les électrodes de mesure (14, 14') et les électrodes de référence (11, 11') peuvent être reliées, deux par deux, aux entrées d'un amplificateur de mesure (3) au moins.

15. Capteur suivant la revendication 14, caractérisé en ce que différents groupes d'électrodes de mesure (14, 14') et d'électrodes de référence (11, 11') peuvent être alimentés par une tension de polarisation inverse.

16. Capteur suivant la revendication 15, caractérisé en ce que les groupes d'électrodes de mesure (14, 14') et d'électrodes de référence (11, 11'), polarisés en inverse, peuvent être reliés aux entrées TEC d'un amplificateur de mesure (3), par l'intermédiaire d'un commutateur (40) inversant la tension de polarisation.

17. Capteur suivant la revendication 15, caractérisé en ce que les groupes d'électrodes de mesure (14, 14') et d'électrodes de référence (11, 11'), polarisés en inverse, peuvent être reliés aux entrées TEC d'un amplificateur de mesure (3, 3'), dont les sorties peuvent être reliées à un circuit d'analyse (38), avec une possibilité de permutation.

18. Capteur suivant l'une quelconque des revendications 6 à 17, caractérisé en ce que l'électrode de mesure (14), enduite de l'enzyme, peut être alimentée par une tension de polarisation positive, l'électrode de mesure sans enzyme (14') pouvant être alimentée par une tension de polarisation négative, par rapport à l'électrode de référence correspondante (11, 11').

19. Capteur suivant la revendication 18, caractérisé en ce que, dans le cas d'un capteur combiné (1) pour la mesure du glucose et de l'oxygène, l'électrode de mesure en platine (14), enduite de glucose oxydase (20), et l'électrode de mesure en platine (14'), sans enzyme, présentent des tensions de polarisation inverses de 600 à 750 mV, par rapport à leurs électrodes de référence (11, 11') en acier spécial et/ou argent.

20. Appareil pour la mesure in vivo et le contrôle de substances contenues dans des liquides organiques,

telles que $\beta$ - D - glucose et/ou oxygène dans le sang ou liquide tissulaire, avec un capteur à implanter suivant l'une quelconque des revendications 1 à 19, un amplificateur de mesure, recevant le signal du capteur, et un circuit d'analyse du signal de sortie de l'amplificateur de mesure, muni d'un convertisseur analogique/numérique, caractérisé en ce qu'un potentiel de référence au moins est réglable en continu à l'entrée du convertisseur analogique/numérique (50), pour l'étalonnage du signal de sortie analogique de l'amplificateur de mesure (3), par une adaptation à des valeurs prédéfinies d'un fluide d'essai, en ce que les valeurs de mesure numériques, ainsi étalonnées, peuvent être mémorisées dans une mémoire à lecture-écriture (60), à intervalles prédéfinis, en ce que le contenu de la mémoire à lecture-écriture (60) peut être extrait par l'intermédiaire d'une interface (70), à intervalles plus importants, et être transmis à une imprimante et/ou à un ordinateur externe, et en ce que le circuit de mesure et de mémorisation est réalisé sous forme de montage portatif fonctionnant sur piles, dans la technique CMOS à faible consommation de courant.

**21.** Appareil suivant la revendication 20, caractérisé en ce qu'un affichage à cristaux liquides (52) est prévu en supplément, pour afficher les valeurs de mesure et de service instantanées.

**22.** Appareil suivant l'une des revendications 20 et 21, caractérisé en ce que les entrées de l'amplificateur de mesure (3), qui peuvent être alimentées par une tension de polarisation et raccordées aux électrodes (11, 11', 14, 14') du capteur électrochimique (1), ont une polarité réversible.

**23.** Appareil suivant l'une des revendications 20 et 21, caractérisé par deux amplificateurs de mesure (3, 3'), reliés à différentes cellules partielles du capteur électrochimique, et dont les entrées sont alimentées par une tension de polarisation réglable, indépendamment l'une de l'autre, les sorties pouvant être reliées, d une manière sélective, à l'entrée analogique du convertisseur analogique/numérique (50).

**24.** Appareil suivant l'une quelconque des revendications 20 à 23, caractérisé en ce que le circuit d'analyse (38) comporte un microprocesseur (80) alimenté par les valeurs de mesure et/ou de service de l'appareil, et permettant de déclencher des signaux d'alarme et/ou l'apport d'agents pharmaceutiques, suivant la concordance et/ou l'écart des valeurs de mesure instantanées avec et/ou par rapport à une valeur de consigne au moins et/ou une courbe de consigne au moins, et/ou suivant les valeurs de service.

**25.** Appareil suivant la revendication 24, caractérisé en ce que le microprocesseur (80) comporte un programmme d'attaque d'une pompe à insuline (82), suivant l'écart entre les taux de glucose mesurés dans le sang ou dans les tissus et une valeur de consigne ou une courbe de consigne au moins, et en ce que les quantités d'insuline, déterminées par le programme, peuvent être mémorisées dans une mémoire à lecture-écriture (60), avec les valeurs de mesure du glucose, et être appelées par l'interface.

Fig. 3

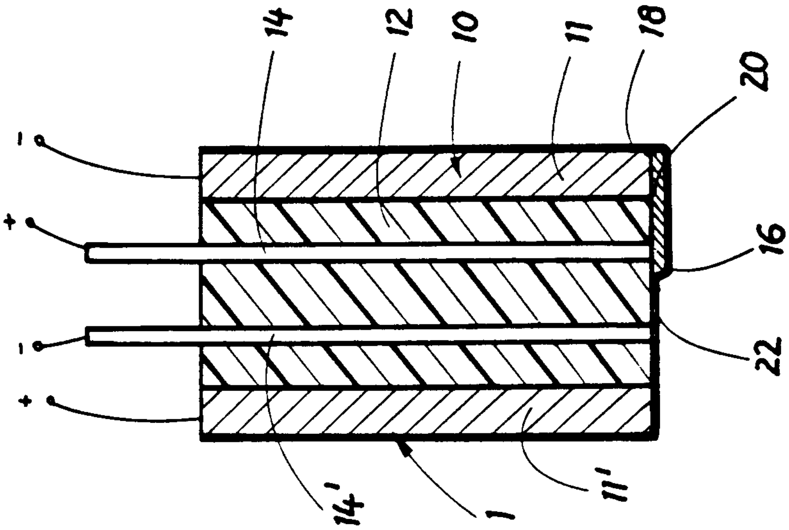


Fig. 2

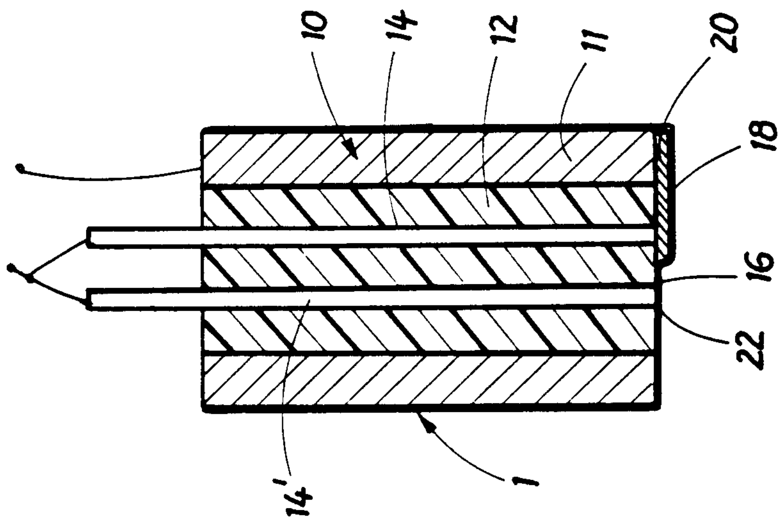


Fig. 1

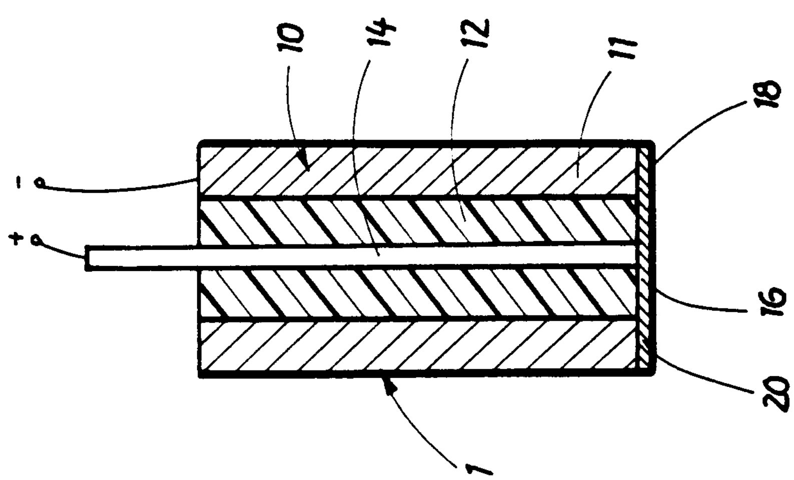

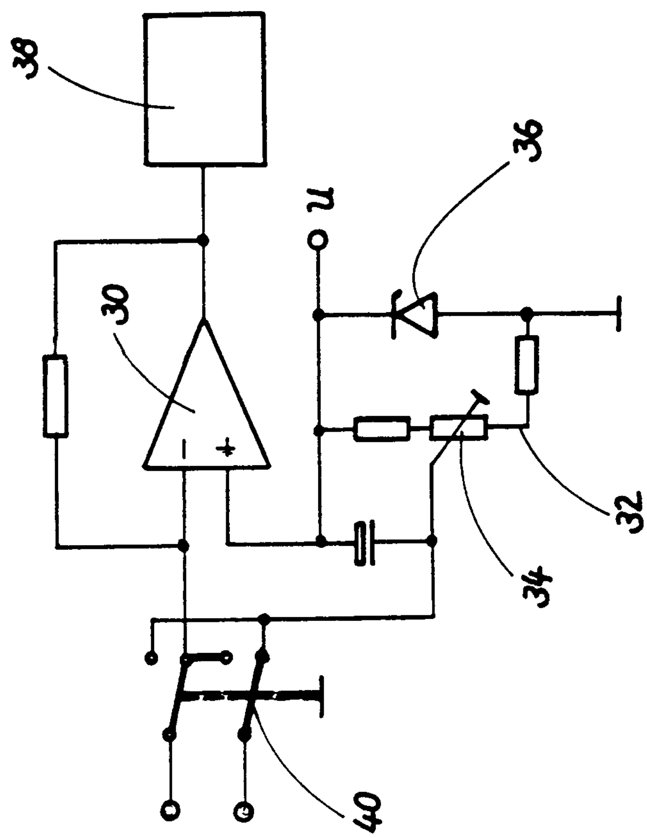


Fig. 5

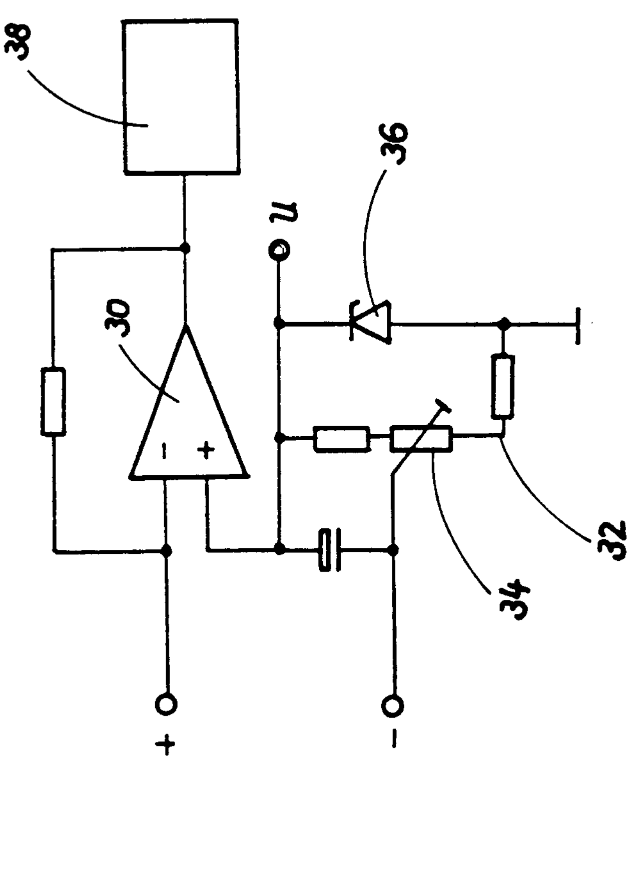


Fig. 4

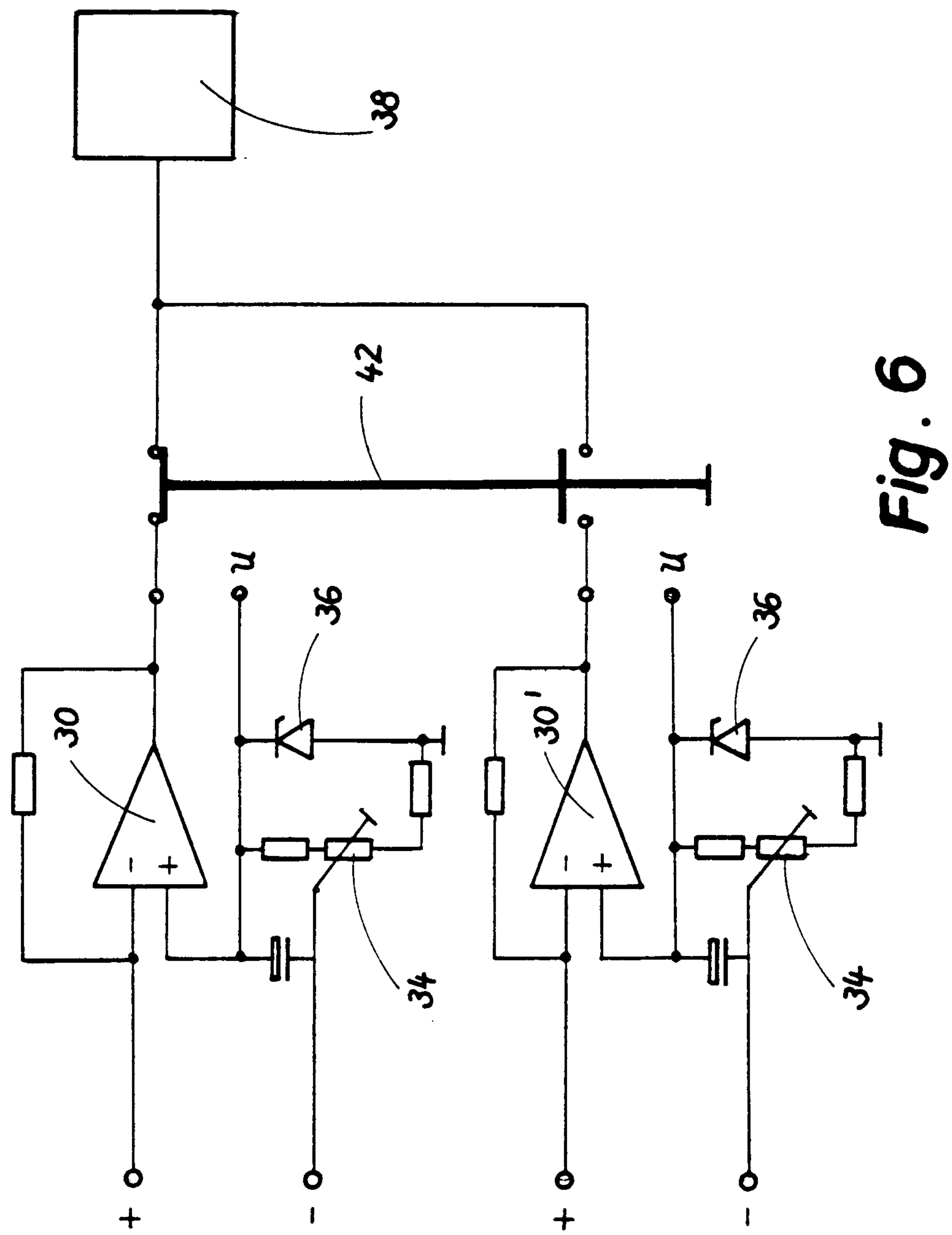


Fig. 6

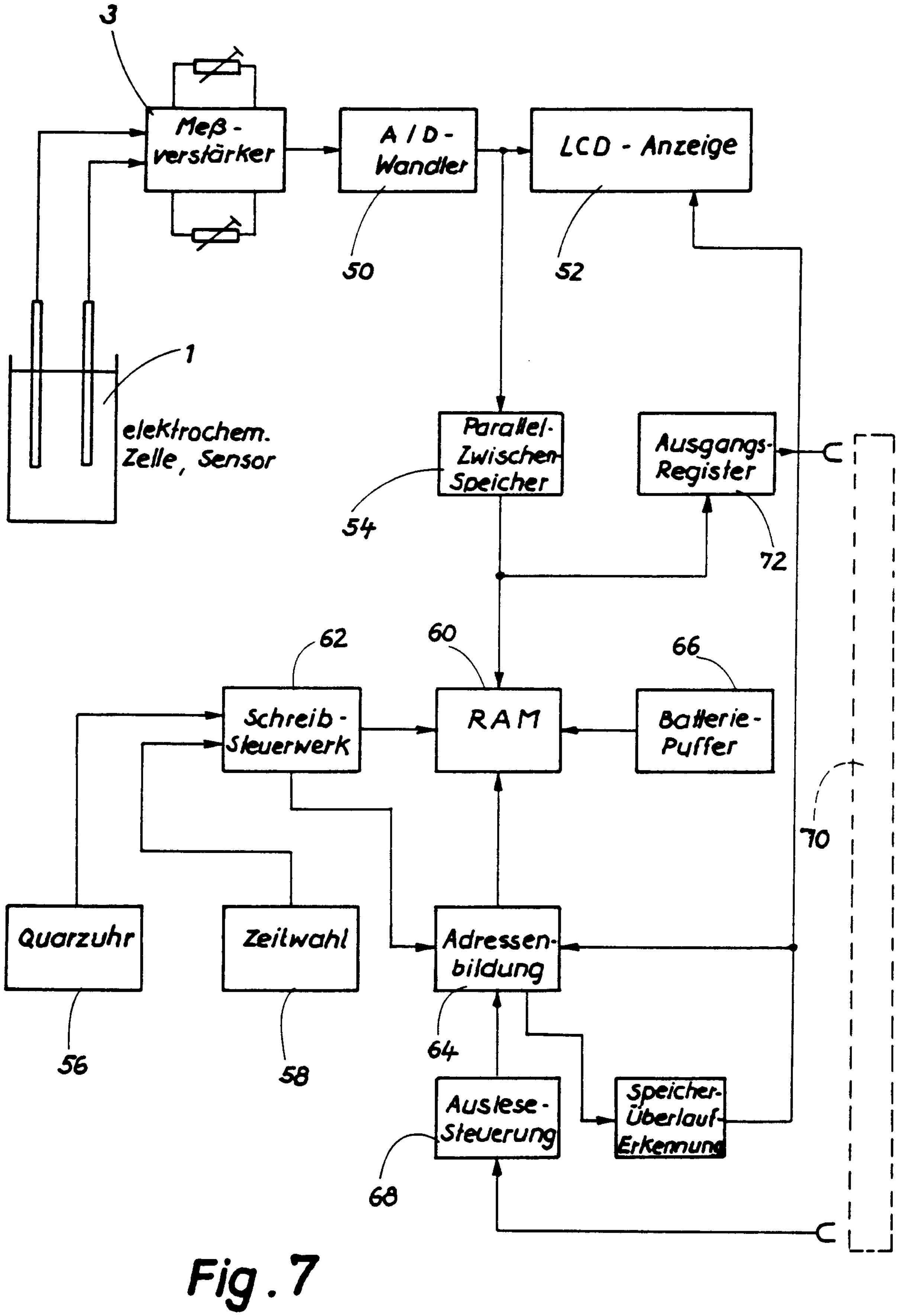
3
Meß-
verstärker
A / D-
Wandler
LCD - Anzeige
50
52
1
elektrochem.
Zelle, Sensor
Parallel-
Zwischen-
Speicher
Ausgangs-
Register
54
72
62
60
66
Schreib-
Steuerwerk
RAM
Batterie-
Puffer
70
Quarzuhr
Zeitwahl
Adressen-
bildung
56
58
64
Auslese-
Steuerung
Speicher-
Überlauf-
Erkennung
68

Fig. 7

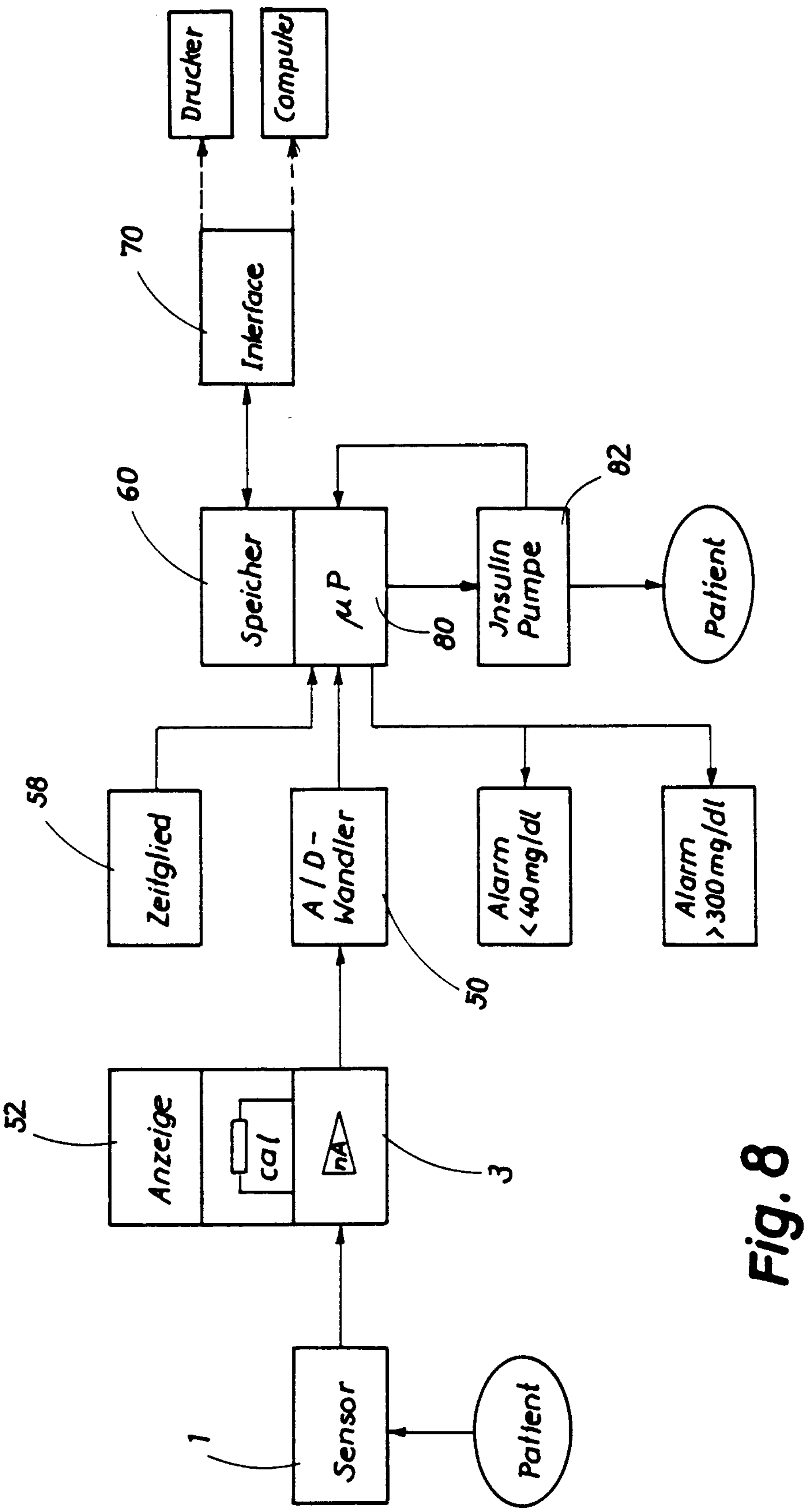
Drucker
Computer
70
Interface
60
Speicher
μP
80
82
Insulin Pumpe
Patient
58
Zeitglied
A/D-Wandler
50
Alarm <40 mg/dl
Alarm >300 mg/dl
52
Anzeige
cal
nA
3
1
Sensor
Patient

Fig. 8